# EUROPEAN PATENT APPLICATION

(11) **EP 3 437 604 A1**
(43) Date of publication of application: **06.02.2019**
(21) Application number: 18187529.5
(22) Date of filing: 06.08.2018
(51) Int. Cl.: A61F 13/49, A61F 13/494, A61F 13/505

(54) **GARMENT WITH MOISTURE MANAGEMENT INCONTINENCE PAD**

(30) Priority: 04.08.2017 WO PCT/NL2017/050524
(71) Applicant: D.G. Plastex B.V., 5111 PS Baarle-Nassau (NL)
(72) Inventor: Gielis, Peter Paul, 5111 PS Baarle-Nassau (NL); Derboven, Sandy Emile Nicole, 5111 PS Baarle-Nassau (NL)
(74) Representative: V.O.

(57) **Abstract**

The present invention relates to a garment comprising a moisture management incontinence pad (100) adapted for use within the garment (5), wherein the pad comprises: (a) at least one sheet of a liquid absorbent material (1) ; (b) a first (10) and a second (20) inside sheet of a liquid impermeable material, positioned at the opposite first and second opposite end sides (8, 9) of the pad (100) ; (c) at least one third sheet (30) of a liquid impermeable material, which extends in longitudinal direction of the pad and is positioned on a side of the liquid absorbent material (1) proximal to the garment ; (d) a fourth outside sheet of a liquid impermeable material (40), which is positioned on an outside face (35, 21, 22) of the first, second and third sheet of liquid impermeable material (10, 20, 30) which is provided to face the garment.

## Description

The present invention relates to a garment, in particular an undergarment, more in particular underpants, having a moisture management incontinence pad attached therein, and to a method for producing such a garment and moisture management incontinence pad.

Moisture management incontinence pads and undergarments containing such moisture management pads are known in the art, and commercial products have been developed for use with male as well as female individuals, adults as well as children. Incontinence is namely not limited to babies or children who not yet learned how to be clean. Incontinence additionally is not necessarily linked to the age of a human being and it includes but is not limited to urinary incontinence. In fact, incontinence may occur at various locations of the human body. As an example, it is believed that one woman out of three suffers from involuntary leakage of liquid from the body, including breast leakage as well as urinary incontinence.

Incontinence may find its origin with a wide variety of causes. Urinary incontinence may for example be caused by medication. Male incontinence may be caused by health conditions including prostate surgery, neurologic disease or injury, such as Parkinson's disease, stroke or spinal cord injury; it may be due to obstructed urination and certain birth defects or chronic medical conditions such as diabetes. Post-micturition dribble is a condition, which involves involuntary loss of urine after finishing passing urine by a male individual, and cloths have been re-arranged, which may cause trousers to get wet. Female incontinence may involve periods of urinary incontinence caused by childbirth and menopause, but it may also involve leaking of fluid from female breasts in periods of breast-feeding.

Many commercial products exist to manage incontinence, and they address many levels of incontinence. Amongst these a wide variety of disposable products has been made available. Although these products have been optimized in the course of time, they may be sensed as uncomfortable by the person wearing it and cause embarrassment. Besides this, disposable incontinence products may be the cause of many infections, they are expensive and their disposal puts a large burden on the environment. Sanitary towels are usually composed of a series of layers of liquid absorption material, reciprocally joined by seams. Sanitary towels are usually of the disposable type and have a flattened shape so as to be placed between the underwear and the skin of the person wearing the sanitary towel. In this position they hinder movement of the user and thereby determine a low level of comfort. Sanitary towels often comprise an upper layer made of a material suitable to contact the skin and to permit the transit of liquids or moisture, an absorbent layer suitable to absorb the liquids filtering through the upper layer, and a lower layer suitable to come into contact with the garments such as a bra or pair of knickers and prevent the passage of liquid to the garment, thereby preventing staining thereof.

Reusable incontinence pads are known in the art as well. They typically include a moisture absorbing component, which is provided to contact the skin, and an overlying moisture barrier designed to prevent moisture leakage outwardly from the pad. These reusable pads are usually washable and recyclable.

Products of the type described above are of fairly small size and are intended to be worn inside a pair of underpants and, during use, to be held in contact against the body of the user by pressure from the underpants. Fastening means in the form of self-adhesive glue may be present, which allows fastening inside underpants. Other types of fastening means include friction coatings and mechanical fastening means, such as press-studs and hook and loop surfaces.

US2005/273068 discloses a moisture management incontinence pad adapted for placement within an undergarment worn by a user. The incontinence pad comprises a multi-layer fabric composite having an inside major surface for residing nearest the body of the user and an outside major surface for residing nearest the undergarment. The fabric composite comprises a plurality of overlying absorbent layers having respective first and second opposing end edges and first and second opposing side edges. To promote circulation between said overlying layers during laundering, the absorbent layers are unattached to one another along a portion of at least one of respective first and second side edges and first and second end edges. The multi-layer fabric is received in a liquid impermeable jacket, which covers the opposing end edges and opposing side edges of the absorbent layers. The jacket forms a vapor permeable moisture barrier between the composite and the undergarment. The jacket also defines an open moisture entry zone which communicates with a portion of the inside major surface of the fabric composite, whereby moisture is received through the entry zone and into the fabric composite for transport away from the body of the user.

The free end of the fabric composite is releasably attachable inside the pocket of the jacket with buttons, such that the fabric composite is removable from the pocket and extendable outwardly from the jacket for laundering and drying. However, the presence of buttons at the indicated position in underpants is experienced as particularly uncomfortable. Additionally, when used with underpants three separate parts have to be combined, i.e. the moisture management incontinence pad, the pocket and the underpants, which is not user friendly. Besides this, correct positioning of a removable moisture management incontinence pad in underpants in such a way that it will always take an optimal position to absorb moisture and preventing folding or moving of the pad during use, is extremely difficult.

BE1010279 discloses a washable, reusable slip for incontinent individuals, children and adults. The slip is provided with an absorbent part and a liquid-tight layer arranged on the outside thereof. At least in the lower portion of the slip one or more channels of liquid-tight portions are provided, which connect to the liquid-tight layer and the absorbent section. To achieve this, facing edges of strips of opposite liquid tight sheets are folded towards and stitched onto a liquid permeable sheet. The strips of liquid tight sheet preferably comprise a woven material, provided with a water tight coating, on the side facing the absorbent layer.

The product disclosed in BE1010279 presents the disadvantage that liquid can still leak from the liquid absorbent layer towards the remainder of the slip. In particular, leakage risks to occur at the position where the strip is connected to the liquid absorbing layer, because the woven material contacts and is fastened to the liquid absorbing layer.

The present invention seeks to provide a solution to this problem.

In particular, the present invention seeks to provide an under garment containing a moisture management incontinence pad, which garment is re-usable and washable, and shows a reduced risk to the occurrence of leaks from the liquid absorbing layer. The present invention also seeks to provide a moisture management incontinence pad and a method for producing such a garment and a moisture management incontinence pad for use with such a garment.

This is achieved according to the present invention with a garment, which shows the technical features of the characterizing portion of the first claim.

Thereto the present invention relates to a garment comprising a moisture management incontinence pad (100) attached to the garment, wherein the incontinence pad comprises:
(a) at least one sheet of a liquid absorbent material, with an inside surface provided for residing nearest the body of a user of the moisture management incontinence pad and an outside surface provided for residing nearest the undergarment, wherein the sheet of liquid absorbent material comprises a pair of opposite first and second upright end sides s which extend crosswise of the pad, and a pair of opposite first and second upright longitudinal sides;
(b) a first and a second inside sheet of a liquid impermeable material, positioned at the opposite first and second opposite end sides of the pad, wherein a first end flap of each of the first and second inside sheet resides along a part of the inside surface of the sheet of liquid absorbent material which extends along respectively a first and second upright end sides of the pad and is connected to the liquid absorbent material in thickness direction thereof, wherein the first end flap of each of the first and second inside sheet is connected to a second end flap of each of the first and second inside sheet crosswise of the pad, which second end flaps extend along at least part of the first end flap crosswise of the pad, wherein each of the second end flaps is connected to a fastening flap at a position opposite the connection to the first end flap,
(c) at least one third sheet of a liquid impermeable material, which extends in longitudinal direction of the pad and is positioned on a side of the liquid absorbent material proximal to the undergarment, which third sheet of liquid impermeable material is provided extend along at least part of the lower outside surface and the opposite upright sides of the sheet of liquid absorbent material, and further along a first and second part of the inside surface which extend along the opposite first and second upright sides of the sheet of liquid absorbent material by means of a first and second cover flap, which first and second cover flaps are respectively covered by a first and second fold flap which extend over at least part of the first and second cover flaps and which are connected to respectively a first and a second side fastening flap of the third sheet of liquid impermeable material at a position opposite the connection to the first and second cover flaps
(d) a fourth outside sheet of a liquid impermeable material, which is positioned on an outside face of the first, second and third sheet of liquid impermeable material which is provided to face the undergarment, opposite end sides of the fourth sheet comprising a third and a fourth end fastening flap which run along respectively the first and second upright end sides of the liquid absorbent material, and which are connected to respectively the first and second end fastening flap of the first and second sheet of liquid impermeable material in thickness direction of the pad at a position beyond the sheet of the liquid absorbent material, which fourth sheet further comprises a first and a second side fastening flap which are connected to respectively the first and second side fasting flap of the third sheet of liquid impermeable material in thickness direction of the pad at a position beyond the sheet of liquid absorbent material,
(e) wherein the first and second side fastening flap of the third liquid impermeable sheet 30 are respectively connected or fastened or attached to the first and second side fastening flap of the fourth liquid impermeable sheet and to the garment. The attachment or fastening will generally be permanent, to ensure optimal positioning of the pad, improve use and wearing comfort and provide a garment which is suitable for washing as a whole. The garment of this invention is particularly suitable for use with elderly people.

Because in the part of the moisture management incontinence pad that is provided to contact the garment, the liquid absorbing material is fully covered or enveloped by a sheet of liquid impermeable material, so that the liquid absorbing material does not directly contact the garment, direct leaking of liquid that had been absorbed by the liquid absorbing material out of the moisture management incontinence pad and/or to the garment may be prevented or at least the risk to such leaking may be reduced to a minimum. Furthermore, the moisture management incontinence pad is connected to the garment exclusively along opposite sides or edges of the moisture management incontinence pad, which are formed by sheets or layers of liquid impervious material, which at the position of the connection to the garment do not contact the liquid absorbent material and are separated therefrom by an upright extending part of the liquid impervious ma terial.

More importantly, the risk to leaking of liquid along any connections connecting the moisture absorbent material to the liquid impervious sheets, or connecting the liquid impervious sheets to the material of the garment may be reduced to a minimum as well, because the liquid absorbent material is exclusively fastened or connected to the full thickness of inside sheets of liquid impermeable material which may possibly contact the liquid absorbing material, but which do not directly contact the garment, nor do they directly contact the skin of the user of the moisture management incontinence pad. Any inside sheets of liquid impermeable material, or parts thereof, which are connected to the liquid absorbent material, are separated either from the user and/or the garment by an additional sheet or layer of liquid impermeable material.

In particular, the risk to leaking of liquid which has been absorbed by the liquid absorbing material towards the first and second fold flap of the third sheet of liquid impermeable material, which may contact the skin of the individual wearing the pad, may be reduced to a minimum. This may be achieved because the liquid absorbent material is exclusively connected to the cover flaps which are located between the liquid absorbent material and the fold flaps, and therefore contact the inside surface of the liquid absorbent material. Furthermore this may be achieved because the connection does not extend throughout the thickness of the first and second fold flaps, in particular not throughout the entire thickness thereof, which cover at least part of the first and second cover flaps which contact the inside face of the liquid absorbent material. This also holds for the first and second sheet of liquid impermeable material and the first and second end flaps thereof. The fact that the connection does not extend up to the inside surface of the cover flaps which may contact the skin of the user, improves wearing comfort.

In the present invention no connection is established between the liquid absorbing material and an outer face of the outside sheet of liquid impermeable material, which contacts the skin of the user, no connection is established between the liquid absorbing material 1 and an outer face of the outside sheet of liquid impermeable material which contacts the garment, and no connection is established between any of these sheets and a face of an inside sheet which could contact the skin of the user. It has namely been observed that a connection between the liquid absorbing material and the liquid impermeable sheet, serves as a guiding for guiding a liquid flow from the liquid absorbing material along the connection to the surface of the neighboring material. Such a connection thus serves as a source for liquid leakage, especially if the connection is established using needling or stitching and holes are punched which extend through the full thickness of the material, i.e. through the full thickness of the first and second end flaps into the liquid absorbing material, and/or through the full thickness of the first and second cover flaps into the sheet of liquid absorbent material.

Because the connection of the liquid absorbing material to the inside first, second and third sheet of liquid impermeable material, may extend through part of the thickness of these material sheets, but does not extend through the full thickness up to an outside surface of these sheets, the risk to leaking of any liquid along that connection into and throughout the first, second and third sheet of liquid impermeable material, towards a lower side of the incontinence management pad, may be reduced to a minimum. This also improves wearing comfort, as the risk to a seam or connection contacting the skin, may be minimized.

The moisture management incontinence pad of the present invention presents the additional advantage that it shows good shape or form stability, without requiring a connection between the liquid absorbing material and the outside sheet of liquid impermeable material, because of the particular arrangement of the first, second and third sheet of liquid impermeable material and the connection of these sheets to the liquid absorbing material. The fact that opposite sides of the moisture management pad are permanently attached to the garment contributes to maintaining this form stability. This permanent attachment may be achieved according to various methods known to the skilled person, such as needling, stitching, knitting, tufting, welding, using an adhesive etc. An advantage of these methods over the use of buttons, push buttons or engaging ribbons is that they occupy little room and show no protruding parts.

Similarly, any liquid that might have leaked along the connection of the liquid absorbent material to the first and/or second sheet is prevented from leaking or flowing to the garment to which the incontinence management pad is mounted, by the presence of a liquid closed connection which connects the first end flaps and the second end flaps which extend along the first end flaps. In a preferred embodiment, the second end flaps are double folded over the first end flaps. The liquid closed connection or the fold is namely open towards the volume or pocket delimited by the parts of the first and second liquid impermeable sheet which extend along the upright end sides of the liquid adsorbent material and the first and second fastening flaps, and is closed in the direction of the inside surface of the sheet of liquid absorbent material, so that a physical barrier is provided against the flowing of liquid from the liquid absorbent material towards the garment.

In a preferred embodiment, any liquid that might have leaked along the connection of the liquid absorbent material to the third sheet is prevented from leaking or flowing to the garment to which the incontinence management pad is mounted, by the presence of a fold which connects the first and second cover flaps of the third sheet and the first and second fold flap which cover those cover flaps, in particular the first and second fold flap which are double folded thereover. The fold is open towards a volume delimited by the upward extending part of the third sheet which extends along the upright sides of the liquid absorbent material, and is closed in the direction of the inside surface of the sheet of liquid absorbent material.

When used in combination with an undergarment, the moisture management incontinence pad of the present invention may be designed so that it can move with the garment in relation to the user's body, for example in a floating arrangement, so that an optimal positioning can be guaranteed in any position of the body. To achieve that two opposite edges of the moisture management incontinence pad may be fastened to a garment, whereas the remaining edges of the moisture management incontinence pad will not be fastened to the garment. This arrangement will in general facilitate cleaning and drying of the undergarment.

The invention is further elucidated in the appending figures and the figure description given below.

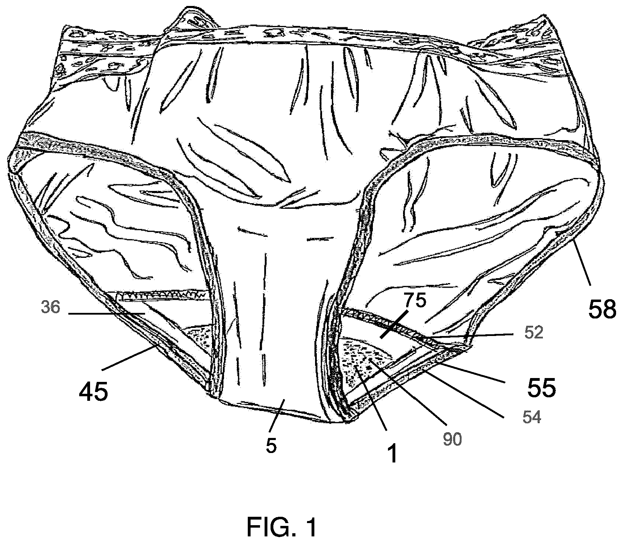

Figure 1 shows a view to an under pant as an example of a garment according to the present invention.
Figure 2 shows a view to the inner part of an under pant according to the present invention, which comprises the moisture management incontinence pad.

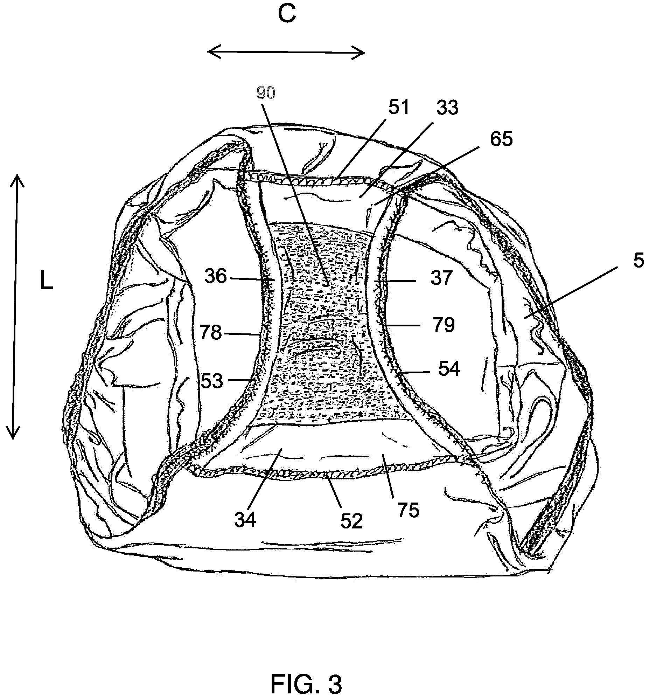

Figure 3 show an enlarged view of a crotch portion of the moisture management incontinence pad in an under garment.
Figure 4A shows a detailed view to a preferred embodiment of a moisture management incontinence pad according to the present invention, with the moisture absorbent pad connected along the leg opening to the garment, and the fold flaps connected to the underlying cover flaps along a continuous connection. In Figure 4B the connection of the fold flaps to the underlying cover flaps is interrupted.
Figure 5 shows a section of the moisture management incontinence pad along the line A-A.
Figure 6 shows a section of the moisture management incontinence pad along the line B-B.
Figure 7 shows the moisture incontinence pad of figure 5, with the upright sides of the liquid absorbent material enveloped by an envelope of a liquid impervious material.
Figure 8 shows the moisture incontinence pad of figure 6, with the upright sides of the liquid absorbent material enveloped by an envelope of a liquid impervious material.

Within the scope of the present invention the wording "liquid" encompasses a wide variety of liquids, which may be polar or apolar, hydrophilic or hydrophobic. In the description below, the wording "liquid" particularly refers to body liquids, which are generally water based or of which the main component is water. Within the scope of the present invention also, the wording "liquid" encompasses "moisture" and vice versa.

The present invention relates to a garment comprising a moisture management incontinence pad 100 attached thereto and is suitable for a wide range of applications, and it may for example be adapted for use within a wide variety of undergarments 5, for example as a sanitary towel, a nappy, a nursing pad bra liner, a female undergarment - for example panties, bikini underwear, thongs, G-strings, swimsuits, nightwear etc. - a male undergarment - for example shorts, boxer shorts, boxer briefs, swimsuits, nightwear, etc. The garment containing the moisture management incontinence pad 100 of the present invention may also be adapted for use within children or adult diapers, in female bra's for example to protect women giving breast feeding, etc. It shall be clear that the garment of the present invention may be used within a wide variety of applications and a wide variety of undergarments, garments and clothing. The presence of the moisture management incontinence pad prevents clothing to be contacted by liquids or moisture excreted by the body, any staining caused by that contact.

The shape of the moisture management incontinence pad 100 of the present invention is not critical to the invention, and a wide variety of shapes may be used. The skilled person will be capable of adapting the shape of the moisture management incontinence pad 100 of the present invention, taking into account the intended use., i.e. the nature of the garment into which it is to be attached. When designed for mounting in underpants, the moisture management incontinence pad 100 will usually take the form of a liner, for example a panty liner. When designed for mounting in a bra, the shape of the moisture management incontinence pad 100 will be adapted to the shape of the bra.

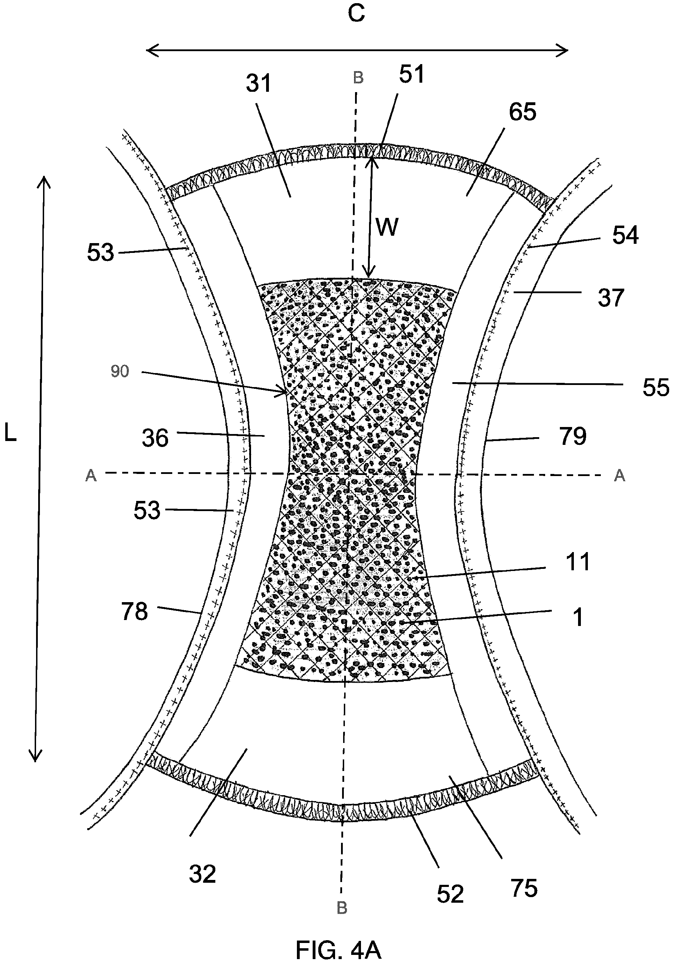

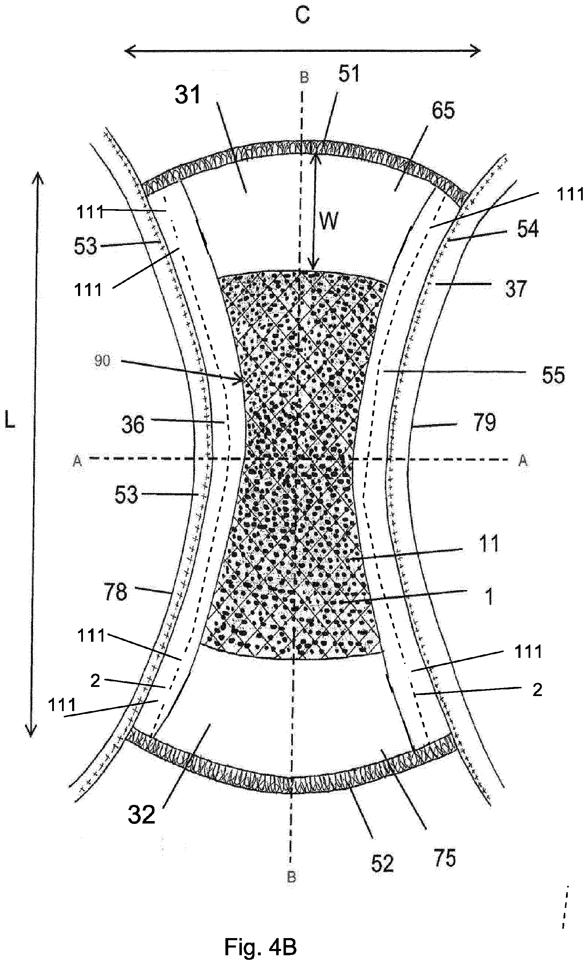

The preferred embodiment of the garment comprising a moisture management incontinence pad 100 shown in figure 4A and B comprises at least one sheet 1 of a liquid absorbent material, with an inside surface 11 provided for residing nearest the body of a user and an outside surface 12 provided for residing nearest the undergarment 5. The sheet of liquid absorbent material further comprises opposite upright end sides 8, 9 in cross direction of the material, and opposite upright sides 6, 7 usually in longitudinal direction of the material, although these may also extend crosswise of the sheet of liquid absorbent material. The inside surface 11 of the sheet of liquid absorbent material is connected to the opposite upright end sides 8, 9 respectively by means of upper end edges 8" and 9', the outside surface 12 of the sheet of liquid absorbent material is connected to the opposite upright end sides 8, 9 respectively by means of lower end edges 8" and 9". The inside surface 11 of the sheet of liquid absorbent material is connected to the opposite upright sides 6, 7 respectively by means of upper end edges 6' and 7', the outside surface 12 of the sheet of liquid absorbent material is connected to the opposite upright sides 6, 7 respectively by means of lower side edges 6" and 7". A strip 90 of the inside surface 11 of the sheet of liquid absorbent material 1, provided for positioning nearest the user to permit absorption of liquid from the user, is accessible and available for acquisition of liquid or liquid absorption. It shall be clear that to achieve this, the strip 90 may be uncovered, or covered with a material which permits transfer of liquid towards the liquid absorbent material 1.

The moisture management incontinence pad 100 further comprises:
- a first and a second inside sheet 10, 20 of a liquid impermeable material. Part of the first and second inside sheet 10, 20 extends in cross direction of the liquid absorbing material, on a side thereof which points away from the side proximal to the person wearing the pad, and along the opposite upright end sides 8, 9 of the liquid impermeable material.
- a third sheet of a liquid impermeable material 30, which extends in longitudinal direction of the pad, i.e. is provided to extend along and between the bows where the garment is an underpants and is positioned on a side 12 of the liquid absorbent material 1, or in other words a side 12 distal from the person wearing the pad, or in other words a side provided to reside proximal to the underpants 5
- a fourth cover sheet of a liquid impermeable material 40, which is positioned on a side 3, 73 of the first, second and third inside sheet distal from the liquid absorbent material 1, or in other words a side which is provided to reside proximal to or is provided to face the undergarment 100.

If so desired, the inside surface 11 of the sheet of liquid absorbent material 1 provided for residing nearest the body of a user may be covered with or may comprise a one way layer of a liquid permeable material 15, which permits and even facilitates absorption of any liquid or moisture contacting the surface of the pad and ensures quick transfer of the absorbed liquid towards the liquid absorbing material 1. Layer 15 may for example act as a liquid acquisition and distribution layer and may be provided to collect and transfer liquid from the person wearing the pad to the lower sheet of liquid absorbent material 1, to ensure that the individual wearing the pad experiences a dry feeling, even after liquid or moisture expelled by that individual has been absorbed by the moisture management incontinence pad. Layer 15 may be made of a material which prevents back flow of liquid or moisture from the liquid absorbent layer back to the skin of the individual wearing the pad. Layer 15 may for example be made of a soft material as it may contact the skin of the wearer. Layer 15 may for example be made of a fleece material, however, any other material considered suitable by the skilled person may be used, such as natural, biodegradable fibre (usually starch fibre or cotton) permitting washing and thereby re-utilisation of the garment.

Layer 15 may also be made of or include a material having anti-bacterial properties and/or it may be made of a material which has been coated or impregnated with an antibacterial substance, for example one or more compounds selected from silver, a silver compound, titanium dioxide, a quaternary silane, hydrogen peroxide, triclosan or zinc pyrithione. The layer 15 may further comprise a substance that combats odour, for example high surface area mineral compositions.

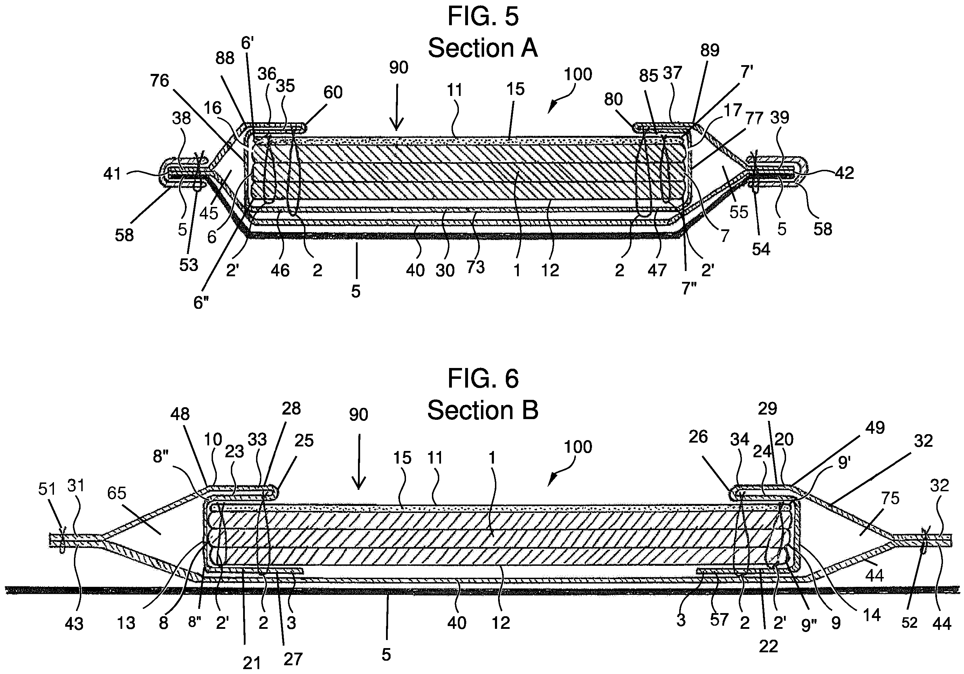

The sheet of liquid absorbent material 1 may be made of any material considered suitable by the skilled person for absorbing liquids in general or body liquids in particular. The sheet of liquid absorbent material 1 may be made of a single layer of liquid absorbent material, or it may be a laminate of two or more layers as is shown in fig. 5 and 6, wherein several layers are combined to form the liquid absorbent material. The individual layers forming the liquid absorbent material may be made of the same material, or of different materials. Where use is made of a laminate, the individual layers may adhere to each other over their entire contact surface, or along part of the contact surface only.

Examples of frequently used liquid absorbent materials include bound, non-woven fibre cloth, so-called non-woven materials, woven materials, perforated plastic films, nets as well as open-cell or perforated foam material. Other suitable materials include natural, highly biodegradable fibre (usually starch fibre or cotton) permitting washing and thereby re-utilisation of the sanitary towel. Laminates, for example laminates of non-woven material and plastic film, can also be used as long as they are capable of absorbing the liquid or moisture. Suitable materials for producing the liquid absorbent material 1 include one or more of the group consisting of polyamide, polyester, polyolefin, polyurethane, polyacrylonitrile, natural cellulose, regenerated cellulose, regenerated cellulose derivatives (i.e. cellulose acetate and cellulose triacetates), natural protein and regenerated protein. The layer of the liquid absorbent material 1 may be produced using technologies such as knitting (warp knitting such as raschel Tricot, weft knitting such as circular or flat), weaving, non-woven methods (blow spinning, staple nonwoven, spun laid, air-laid, needle punched, thermal bonded, hydro-entangled, chemical bonded and so forth), electro-spinning, force-spinning etc.

Additionally the material of which the layer of the liquid absorbent material 1 is made may also include one or more coatings, treatments encapsulation or entrapments, which would enhance its liquid and moisture management functionality, such as rate of absorbency/wicking, absorption capacity, rate of spreading and distribution, one way liquid or moisture transport etc. If so desired, the sheet of liquid absorbent material 1 or one or more layers forming that sheet 1 may be made of or comprise a super-absorbent material, this is a liquid absorbent material capable of absorbing an amount of liquid which corresponds to many times the weight of the super-absorbent material. A number of different types of super-absorbent material is known, and they occur in various different physical forms, such as particles, granules, fibres, in the form of non-woven material, as sheets or as foam. Any type whatsoever of super-absorbent and any physical form whatever of it which is found to be suitable for the purpose can be used within the scope of the invention. The sheet 1 of liquid absorbent material may further comprise a substance that combats odour, for example high surface area mineral compositions.

The liquid absorbing material 1 of the pad 100 and of course the pad 100 in its entirety, may be formed with a dedicated three-dimensional shape which is adapted to at least partially, but preferably fully contact or surround the body part suffering from liquid leak, for example the genitals, or a breast if the pad is intended for use by female users. Thereby the pad and the moisture absorbent material may be given a shape which shows optimal fit with the shape of the body part to be covered.

The first, second, third sheets of liquid impermeable material 10, 20, 30 may be made of the same material, or of different materials. Preferably however they are made of the same liquid impermeable material as this facilitates production of the pad. However, if the pad is intended for a particular application, where particular properties are envisaged, the nature of the material used to make the liquid impermeable sheets 10, 20, 30 may be selected to take into account the intended function. The sheets of liquid impermeable material 10, 20, 30 may comprise, consist of or include any fully or partly liquid-blocking, liquid repelling, moisture blocking or moisture repelling material known to the skilled person, in order that the pad may feel airy and comfortable to wear, to be breathable in dry form, while providing an effective barrier material upon exposure to liquid. In particular, the sheets of liquid impermeable material may be made of a material which is water or moisture repellent, or is coated with a coating providing such properties. Thereby, preferably at least the side provided to face the user is water repellent, but preferably both opposite sides of the sheets of liquid impermeable material are water repellent. This permits minimizing the risk that those parts of the moisture management pad of this invention which are provided to be fastened and/or connected to the material of a garment, would absorb body liquid and function as a guiding for guiding a flow of absorbed body liquid to the garment, and would cause wetting of cloths or other materials which contact the moisture management incontinence pad of this invention.

Furthermore, a material may used which enables the pad to dry more quickly than the use of a liquid impermeable polymer such as a thermoplastic polyurethane film. The liquid impermeable sheet 10, 20, 30 may be made of a liquid impermeable polymer (e.g. a thermoplastic polyurethane film), a liquid permeable or impermeable material coated with a hydrophobising material, for example a lightweight tightly knitted/woven fabric coated with SAP/hydrogel, or the liquid impermeable material may be a lightweight tightly knitted/woven fabric made using textile/SAP hybrid fibres. Alternatively, the liquid impermeable material may be a liquid-proof membrane, or it may be made of a thin plastic film, or of a non-woven material which is made liquid impermeable by coating or treatment with a liquid resistent material, or of a plastic foam with closed cells, different kinds of liquid barrier laminates etc.

The moisture management incontinence pad 100 comprises a first and a second inside sheet 10, 20 of a liquid impermeable material.

The first inside sheet 10 is provided to extend along, preferably to envelope
1. at least a part of the outside surface 12 of the liquid absorbing material which extend along a lower edge 8" of upright end side 8'
2. upright first end side 8 of the liquid absorbing material,
3. at least part of the inside surface 11 of the liquid absorbing material which extends along an upper edge 8' of upright end side 8.

Similarly, second inside sheet 20 is provided to extend along or to envelope
1. at least a part of the outside surface 12 of the liquid absorbing material which extends along a lower edge 9" of upright end side 9
2. upright second end side 9 of the liquid absorbing material, on a side of the liquid absorbing material 1 opposite the upright first end side 8,
3. at least part of the inside surface 11 of the liquid absorbing material which extends along an upper edge 9' of upright end side 9 opposite end side 8.

To achieve the above, at least a part 27, 57 of the first and second inside sheet 10, 20 is positioned and extends proximal to the outside surface 12 of the liquid absorbing material 1, i.e. the side of the liquid absorbing material pointing towards the undergarment.

To achieve the above, further, a first end flap 23 of the first inside sheet 10 is provided to extend mainly along the inside surface 11, in particular to reside on the inside surface 11, in a direction which extends along at least part of, but preferably along the entire length of the upright end side 8. A first end flap 24 of the second inside sheet 10 is provided to extend mainly along the inside surface 11, in particular to reside on inside surface 11, in a direction which extends along at least part of, but preferably along the entire length of upright end edge 9 of the sheet of liquid absorbent material 1. The first inside sheet 10 further preferably comprises a lower flap 21, which is provided to extend along the outside surface 12, in particular along the part of the outside surface 12 which runs along upright end edge 8 of the sheet of liquid absorbent material 1. Similarly, the second inside sheet 20 further preferably comprises a lower flap 22, which is provided to extend along the outside surface 12, in particular along the part of the outside surface 12 which runs along upright end edge 9 of the sheet of liquid absorbent material 1.

Thus, according to the present invention the first and second inside sheet 10, 20 may extend along the upright end sides 8, 9 of the liquid absorbing material or not, they may be made and used as separate sheets, which extend along part of the inside surface 11 of the liquid absorbent material and along part of the opposite outside surface 12 of the liquid absorbent material as described above. Lower flaps 21, 22 may be made as separate parts which are connected to the sheets 10, 20 along the lower end edges 8" and 9" of the liquid absorbent material. In that case, preferably the connection is liquid tight, to minimize the risk to leaking of liquid past the connection. According to an alternative embodiment, the first and second inside sheet 10, 20 may be made in one part and arranged in such a way that they are connected to each other along the outside surface of the liquid absorbent material, i.e. the outside surface of the liquid absorbent material proximal to the undergarment. Making the first and second sheet 10, 20 in one part may increase the material thickness of the moisture management incontinence pad, and this may be desirable for some applications but may be undesired in other applications as well. On the other hand, making the first and second sheet 10, 20 in one part presents the advantage that the risk to liquid leaking into end pockets 65, 75 and further to the connection 51, 52 of the fourth sheet 40 and the first and second sheet 10, 20 is minimal.

The first end flap 23 of the first inside sheet 10 is connected to a second end flap 33 of the first inside sheet, the first end flap 24 of the second inside sheet 20 is connected to a second end flap 34 of the second inside sheet. Thereby, the second end flap 33, 34 may be made in one part with the first end flap 23, 24 or the flaps are made as separate parts which are connected to each other, preferably by means of a liquid tight connection. The connection may for example be a mechanical connection, such as needling, stitching, braiding, stapling, glueing, welding, or any other equivalent connection technique considered suitable by the skilled person, which may be established in a liquid tight manner. The second end flap 33 covers, or in a preferred embodiment is double folded along a fold 25, over at least part of the first end flap 23 in cross direction of the incontinence pad 100 in the direction of the first upright end edge 8 of the liquid absorbing material. On a side opposite the fold 25, the second end flap 33 is connected to a first fastening flap 31 of the first inside sheet 10.

The second end flap 34 covers, or in a preferred embodiment is double folded along a fold 26, over at least part of the first end flap 24 in the direction of the second upright end edge 14 of the liquid absorbing material. On a side opposite the fold 26, the second end flap is connected to a second fastening flap 32 of the first inside sheet 10. The first and second fastening flaps 31, 32 serve to connect the first and second inside sheet 10, 20 to respectively a third and a fourth end fastening flap 43, 44 on opposite end sides of the fourth sheet of liquid impermeable material, which is provided to form a liquid tight shield towards the undergarment.

The first end flap 23 and second end flap 33 on the one hand, and first end flap 24 and second end flap 34 on the other hand may be formed in one part and connected to each other by a fold, as is shown in figure 6. Thereby, second end flap 33 is folded with respect to first end flap 23 in a direction which points towards upright end edge 8 of the liquid absorbing material, the fold being located at the position of the inside surface 11 of the liquid absorbing material 1. Thereby also, second end flap 34 is folded with respect to first end flap 24 in a direction which points towards upright end edge 9 of the liquid absorbing material, the fold being located at the position of the inside surface 11 of the liquid absorbing material.

Both the first end flap 23 of the first inside sheet 10, and the first end flap 24 of the second inside sheet 20 are connected 2 to the liquid absorbent material 1, in thickness direction of the liquid absorbent material. Preferably the connection 2 extends through the entire thickness of each of the end flaps 23, 24 and over at least part of the thickness of the liquid absorbent material, but the connection will preferably extend over the entire thickness of the liquid absorbent material 1. The connection 2 will generally not extend through the entire thickness of second end flaps 33, 34 up to the outside face 28, 29 of respectively the end flap 33, 34 provided to reside proximal to the individual wearing the moisture incontinence pad of this invention. Preferably, the connection 2 will not extend towards, or into or through the second end flaps 33, 34. The absence of a connection of the liquid absorbent material and the second end flaps 33, 34 as described above, which reside on a side of the incontinence pad provided to face the individual wearing the undergarment with the moisture incontinence pad, ensures that the risk to leaking of liquid from the liquid absorbent material 1 towards the second end flaps 33, 34 and face 28, 29 provided to reside proximal to the individual wearing the moisture incontinence pad is minimal. The inventors have namely observed that connections, for example in the form of stitching, needling, braiding, stapling, function as a transport means for leaking liquid and promote leaking of liquid along them. Moreover, because the fold 25, 26 is closed towards the part of the surface of the liquid absorbing material which will contact the person wearing it, any liquid that may leak along the connection between the liquid absorbing material and the first end flaps 23, 24 may be collected in an end pocket 65, 75 which is entirely delimited by liquid impermeable material.

Where the first and second sheet 10, 20 respectively comprise lower flaps 21, 22 which extend crosswise of connection 2, the connection 2 to the liquid absorbent material, may also extent towards and into those lower flaps 21, 22. This way form stability of the moisture management incontinence pad may be ensured, and maintenance of a desired positioning of the first and second sheet 10, 20 with respect to the liquid absorbent material.

The connection 2, 2' will usually not only serve to connect flap 35 and 85 to the moisture absorbent material, but may also connect flap 35 and 85 to respectively flap 33 and 34 at the position where flap 35 overlaps with flap 33, 34, and at the position where flap 85 overlaps with flaps 33, 34. Along connection 2, 2' additionally, flaps 35 and 85 may be connected to respectively flaps 21 and 22, 43 and 44, and 401 and 402. The connection 2, 2' will generally extend between opposite edges 51 and 52 of the moisture incontinence pad. The connection 2, 2' may be a continuous connection. Preferably however, connection 2, 2' is interrupted at the position of flaps 34 and 33 as is shown in figure 4B, wherein one or more interruptions 111 may be provided to minimize the risk that connection 2, 2' would function as a conductor for moisture from moisture absorbent layer 11 to flaps 33 and 34 and further to the undergarment.

In summary, the first sheet of liquid impermeable material 10 is provided to envelope a first end side of the liquid absorbent material as well as part of the adjoining inside and outside surface 11, 12, whereas the second sheet 20 is provided to envelope an opposite second end side of the liquid absorbent material as well as a part of the adjoining inside and outside surface 11, 12.

The moisture management incontinence pad 100 further comprises a third sheet of a liquid impermeable material 30, which extends in longitudinal direction of the pad 100. The third sheet of liquid impermeable material 30 is provided to run along at least part of the outside surface 12 of the sheet of liquid absorbent material 1. The third sheet of liquid impermeable material 30 is provided to also run along opposite upright sides 6, 7 of the sheet of liquid absorbent material 1. The third sheet of liquid impermeable material 30 is further provided to run along a part of the inside surface 11 of the liquid impermeable material 30 which extends along the upright side edges 6, 7 of the liquid absorbent material 1. Where the undergarment is an underpants or slip, the side edges will generally extend along the so-called bows 78, 79 of the underpants or slip. The bows may be provided with an additional reinforcement 58 as they are prone to wearing because of the frequent skin contact.

The part 46, 47 of the third sheet 30 which extends along the outside surface 12 of the liquid absorbent material is connected to the liquid absorbent material 1 and to the parts 76, 77 of the third sheet 30 which extend along the upright opposite sides 6, 7of the liquid absorbent material 1.

Cover flaps 35, 85 of the third sheet 30 are covered by respectively a first and second fold flap 36, 37, in a direction which extends towards the inside surface 11 and runs along the upright sides 6, 7. In a preferred embodiment, the third sheet is made in one part and cover flaps 35, 85 of the third sheet 30 are double folded along a fold 60, 80 to form fold flaps 36, 37. The fold 60 connecting part 35 to first fold flap 36 will generally extend along upright side 6. Similarly, the fold 80 connecting part 85 to first fold flap 37 on an opposite side of the pad 100 will generally extend along upright side 7. First and second fold flap 36, 37 extend over a part of the inside surface 11 and point towards one another, they run along the longitudinal edges 16, 17 of the inside surface 11. On a side opposite the connection to the cover flaps 35, 85, the first and second fold flap 36, 37 are connected to respectively a first and a second side fastening flap 38, 39 of the third sheet 30 along which the moisture management incontinence pad may be fastened to a under garment (see fig. 5). The side fastening flaps 38, 39 extend from respectively the first and second fold flaps 36, 37 in a direction which points away from the first and second upright sides 6, 7 of the liquid absorbent material. When the under garment is an underpants, the opposite longitudinal sides 6, 7 will often be formed by the opposite bows 78, 79 for accommodating respectively the left and right leg of the individual wearing the pant.

The moisture management incontinence pad 100 also comprises a fourth sheet of a liquid impermeable material 40, which is positioned on a side of the third inside sheet 30 which points away from the liquid absorbent material 1,or in other words on the side of the third sheet of liquid impermeable material provided for positioning proximal to an undergarment. The fourth sheet 40 comprises a first and a second end fastening flap 43, 44 which are connected to respectively the first and second end fastening flap 31, 32 of the first and second inside liquid impermeable sheet 10, 20 at a position which beyond the liquid absorbent material 1. The fourth sheet of liquid impermeable material further comprises a first and a second lower side fastening flap 41, 42, which are connected to respectively the first and second side fasting flap 38, 39 of the third inside impermeable sheet 30 at a position which beyond the liquid absorbent material.

The moisture management incontinence pad shows a first and second side pocket 45, 55 or side channel, which extend along the opposite longitudinal sides of the moisture management incontinence pad 100, and first and second end pockets or end channels 65, 75 along the opposite end edges of the moisture management incontinence pad 100. These pockets or channels may serve as a volume capable of storing liquid or moisture that may have leaked from the liquid absorbent material 1 through the liquid impermeable sheets 10, 20, 30 enveloping the liquid absorbent material. The side pockets or channels 45, 55 thus provide an additional barrier between the upright longitudinal sides 6, 7 of the liquid absorbent material and the connection 53, 54 of the third and fourth sheet of liquid impermeable material 30, 40, in the sense that they serve to accommodated an liquid that would have leaked through the upright flaps 76, 77 of the third sheet of liquid impermeable material 30. The presence of this liquid storage space 45, 55 may further minimize the risk of liquid leakage when at the position 53, 54 where the third and fourth sheet of liquid impermeable material are connected to each other, the third and fourth sheet 30, 40 are further also connected to the undergarment, as is shown in figure 5.

The first side pocket 45 is formed by respectively the upright side flap 76 of the third inside sheet 30 of liquid impermeable material, which extends along the upright side 6 of the moisture absorbing material 1, the first fold flap 36 of the third liquid impermeable sheet and the first lower side fastening flap 41 of the fourth liquid impermeable sheet 40. The second side pocket 55 is formed by respectively the upright side flap 77 of the third inside sheet 30 of liquid impermeable material, which extends along the upright extending side 7 of the moisture absorbing material 1, the second fold flap 37 of the third sheet and the second lower side fastening flap 42 of the fourth liquid impermeable sheet 40.

The first end pocket 65 is formed by respectively the upright extending part 71 of the first inside sheet 10 of liquid impermeable material, which extends along the upright extending end edge 13 of the liquid or moisture absorbing material, the third end fastening flap 43 of the fourth sheet of moisture impermeable material 40, and the first end fastening flap 31 of the first sheet 10 of moisture impermeable material. The second end pocket 75 is formed by respectively the upright extending part 72 of the second inside sheet of liquid impermeable material 20, which extends along the upright extending end edge 14 of the moisture absorbing material, the fourth end fastening flap 44 of the fourth sheet of moisture impermeable material 40, and the second end fastening flap 32 of the second sheet 20 of moisture impermeable material.

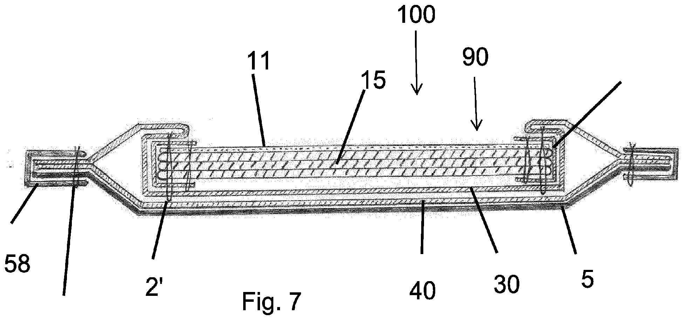

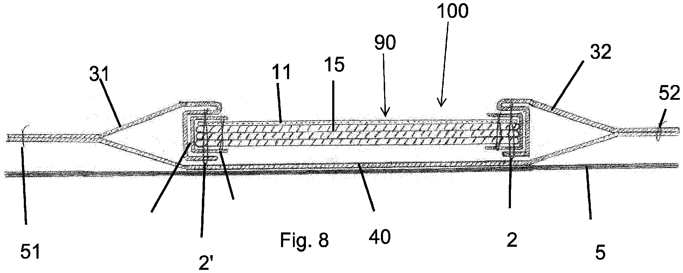

In a preferred embodiment shown in fig. 7, the upright sides 6 and 7 may be enclosed by an envelope at a position between the liquid absorbent material and the upright extending parts of the third liquid impervious sheet 30. In a preferred embodiment shown in fig. 8, the upright sides 8 and 9 may be enclosed by an envelope at a position between the liquid absorbent material and the upright extending parts of respectively the first and second liquid impervious sheet 10, 20. This way the risk to the occurrence of leaks may be further reduced.

It shall be clear that with a different shape of the moisture management incontinence pad and/or of end pockets 65, 75 additional flaps or parts of liquid impermeable sheets 10, 20, 40 may be needed to form end pockets 65, 75. Similarly with a different shape of the moisture management incontinence pad and of side pockets 45, 55 additional flaps or parts of liquid impermeable sheets 30, 40 may be needed to form side pockets 45, 55.

From the description given above, it can be understood that at any position where the sheet of liquid absorbent material 1 may contact material of a garment with which the moisture management incontinence pad is to be used, the liquid absorbent material and garment are always separated by a sheet or a part of a sheet of a liquid impermeable material, so that the risk to direct leakage of liquid or moisture from the liquid absorbent material to the garment is minimal. From the description above it also appears that the parts of the liquid impermeable sheets 10, 20, 30, 40 which may contact the garment, are not connected to or do not contain a connection to the liquid absorbent material 1. To the contrary, at the positions where a connection exists between the liquid absorbent material 1 and a sheet of liquid impermeable material 10, 20, 30, the sheet of liquid impermeable material will always be separated from a garment containing the pad, by a further sheet of liquid impermeable material. The inventors have namely observed that such connections provide a guiding which facilitates and even promotes liquid flow from the liquid absorbent material. Because the liquid absorbing material does not contact these more outwardly positioned sheets of liquid impermeable material, and because no connection exist between the liquid absorbing material and the more outwardly positioned layer of liquid impermeable material, the risk to leaking of liquid absorbed by the liquid absorbing material towards the outside liquid impermeable layer 40 for example along a seam, a connection or any other contact technique, can be reduced to a minimum.

By the use of a liquid, in particular a water repellent material at the position of the first and second fold flaps 36, 37, i.e. the part of the third sheet which extends along the inside surface of the liquid absorbent material and may contact the wearer of the pad, as well as at the position of first and second end flaps 33, 34 of the first and second sheets 10, 20, the risk to the transfer of liquid from the liquid absorbent material 1 to a garment containing the pad, along those flaps may be reduced to a minimum.

When used in a garment, the first and second side fastening flap 41, 42 of the fourth sheet of liquid impermeable material may be used to fasten the incontinence pad of this invention to first and second side edges of the garment 78, 79, positioned on opposite sides of the garment and of the moisture management incontinence pad. However, with a different shape of the garment, for example a bra, fastening may be required along adjacent sides of the moisture management incontinence pad.

According to a preferred embodiment, the first and second end fastening flap 31, 32 are provided to be in a floating arrangement with respect to the garment in which the incontinence pad is to be used. Or in other words, when used in pants, the crosswise extending end edges 31, 32 are not fastened to the material of the underpants. This may facilitate cleaning, washing and drying of the pad when mounted in or fastened to the garment. This will also reduce the risk to visibility of the pad within the cloths worn on top of the under garment and will reduce the risk to tensioning of the under garment at the position of the pad. Moreover, the absence of fastening at that position further reduces the risk to the occurrence of leaks.

When mounted in a garment, in order to ensure that the connection of the incontinence pad to the garment is capable of withstanding wearing which may for example result from friction forces, the first (38) and second (39) side fastening flap of the third liquid impermeable sheet 30 are respectively connected to the first (41) and second (42) side fastening flap of the fourth liquid impermeable sheet and to the garment. Depending on the nature of the garment opposite first and second side fastening flaps 38, 39 may be connected to opposite edges of a garment. In a preferred embodiment the connection may for example extend along a seam 53, 54, which connects inside sheet 30 and outside sheet 40. Where the undergarment is the underpants, opposite first and second side fastening flaps 38, 39 may be connected to an edge of the underpants which extends along the opening in the underpants for receiving a leg. Opposite fastening flaps 31, 32 which extend along cross sides 8, 9 of the pad 1, and when mounted into the underpants extend crosswise between the openings in the underpants for receiving the legs, will generally not be connected to the underpants. Connections 51, 52 generally serve to connect the first and second liquid impermeable sheet to the fourth liquid impermeable sheet, and will not connect the moisture absorbent pad to the underpants. Similarly, in case of other undergarments, the moisture absorbent pad may be connected to the undergarment along two opposite sides or edges, while the other sides or edges are loose from the undergarment. This way of connecting the moisture absorbent pad to the undergarment permits some movement of the pad within the garment which may be needed to permit washing, drying, ironing, and which may correct for any differences in expansion between the material of the undergarment and the pad during washing, drying, ironing or any other use.

However, depending on the nature of the garment, connecting of the moisture management incontinence pad may be preferred along other edges, for example two adjacent edges. In general, only part of the edges or sides of the moisture management incontinence pad will be connected to the garment, to accommodate for different expansion coefficient of the material of the pad and the garment.

In a preferred embodiment, the third sheet 30 and the fourth sheet 40 of liquid impermeable material are fastened to each other on opposite sides of the liquid permeable material 1 along their respective fastening flaps at a position 53, 54 adjacent to and beyond the upright side edges 6, 7 of the liquid permeable material 1. The absence of a contact with and a connection to the liquid permeable material 1 assists in minimizing the risk to leaking of liquid from the liquid permeable material 1 along the connection.

It shall be clear from the description above, that with the pad mounted in a garment, for example an under garment, for example a bra or an underpants, the liquid absorbent material, the liquid impermeable material and the under garment are at no position connected by one and the same connection. This provides a basis for minimizing the risk to liquid leakage.

Connecting or fastening of the liquid impermeable sheets to each other and/or to the material of the garment may be achieved using any technique considered suitable by the skilled person, for example one or more techniques selected from the group of stitching, braiding, tufting, needling, or a combination of two or more of these techniques. However any other technique considered suitable by the skilled person may be used.

The moisture management incontinence pad of the present invention may be made in one part with the afore-mentioned garments. In that case preferably only two opposite sides of the pad are fastened to the garment, whereas the remaining sides of the pad are not fastened to the garment. This will facilitate cleaning and drying of the pad, which may be adviseable because of the moisture absorbent properties of at least part of the material of the pad, and because a large part of the surface of the pad is covered by liquid impermeable material, which will complicate and prolong the time needed to dry. But more importantly, this will contribute to minimizing the risk to the occurrence of liquid leaks from the pad to the garment. The inventors have namely observed that this construction assists in minimizing the occurrence of liquid leaks to the material of the garment 5, especially at the position where for example the first 23, 24 end flaps and the second end flaps 33, 34 of the third sheet of liquid impermeable material are connected to the cover flap 35, 85 and the fold flap 36, 37 of the first and second sheet of liquid impermeable material.

According to another possible embodiment of this invention, the pad 100 is made as a separate part provided for removable mounting in the garment. In that case, the pad preferably comprises fastening means for removably fastening the pad to a garment. Removable fastening may for example be achieved by the use of press studs, wherein one part of the stud is mounted to the pad and the complementary part is mounted to the garment; by the use of co-operating hook- and loopband, wherein one of both is mounted to the pad and the other complementary band is mounted to the garment; co-operating loops and hooks, wherein for example a hook is mounted to the garment and a loop to cooperate therewith is mounted to the pad. It shall be clear that many other removable fastening means known to the skilled person can be used.

The present invention also relates to a garment which comprises one or more moisture management incontinence pads as described above.

The pad of the present invention may be produced as follows.

In a first step the sheet of liquid acquisition material 15 is connected to the sheet of liquid absorbent material 11. The sheet of liquid absorbent material 11 may comprise one single layer or several layers positioned on top of each other. When use is made of a layered material, the sheet of liquid acquisition material 15 is preferably connected to all the layers of the sheet of liquid absorbent material 11. More preferably the connection extends from a top surface 9 of the sheet of liquid acquisition material 15, up to the lower outside surface 12 of the sheet of liquid absorbent material.

In a second step, the first and second sheet of liquid impermeable material 10, 20 are connected to the pad 1. Thereto a part 3, in particular a lower flap of each of the first and second sheet of liquid impermeable material 10, 20 is positioned along the lower crosswise extending edges 8" and 9" of the pad 100, further from those lower edges along the upright sides 8 and 9 towards the upper crosswise extending edges 8' and 9', so that a flap of each of the first and second sheet 23, 24 extends along opposite sides of an upper face 90 of the pad 1. Parts 3, liquid absorbent material 11, preferably liquid acquisition material 15 and flap 23, respectively 24 are connected together. This may for example be achieved by means of a connection which extends through the thickness of these layers. Preferably the connection extends along the length of the pad in cross direction. The first and second sheet are double folded over the flaps 23, 24 to form cover flaps 33, 34. It is remarked that the connection will preferably not extend through the cover flaps 33, 34. The width of the flaps 33, 34 and 23, 24 will be selected such that it is sufficiently small to leave a liquid absorption area 90 along the surface of the pad, for liquid acquisition, and sufficiently large to form a liquid barrier and prevent liquid flow from the liquid absorption area 90 towards the connections 51, 52. Cover flaps 33, 34 are extended to form fastening flaps 31, 32 which extend along upright cross sides 8, 9 of the pad 1. It is remarked that the first and second sheet of liquid impermeable material may be made in one part. In that case, the sheet will extend along the lower outside surface 12 of the pad 1.

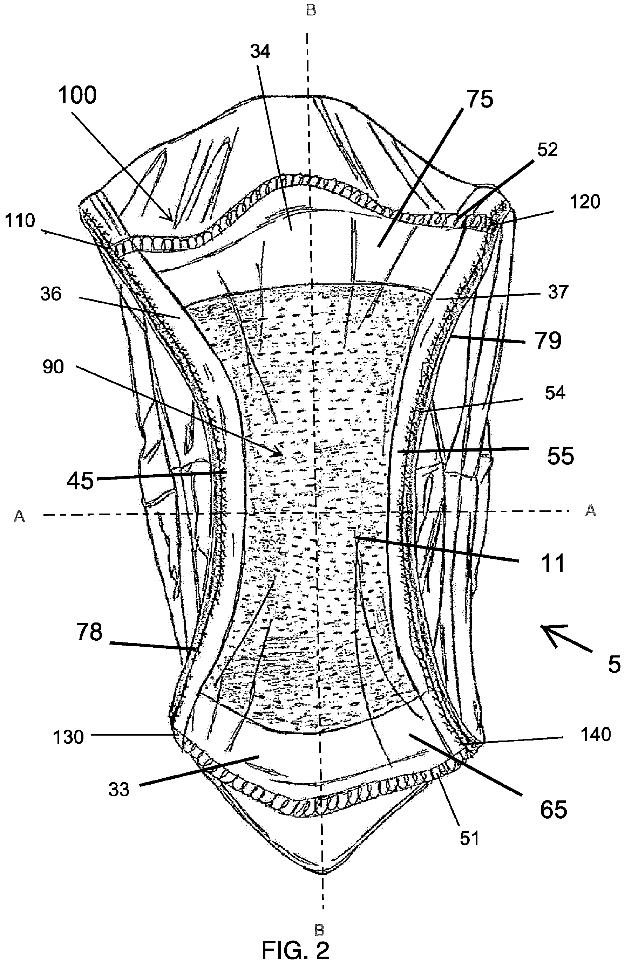

In a third step, the third sheet 30 of liquid impermeable material is positioned along the lower surface 12 of the pad 1 of liquid absorbent material. The third sheet is then folded along the lower edges 6", 7" to extend along the upright longitudinal sides 6, 7 of the pad 1, and further along the upper edges 6', 7' to form flaps 35, 85 which extend along opposite longitudinal sides of the pad, along opposite sides of an upper face 90 of the pad 1. The width of the flaps 35, 85 and 36, 37 will be selected such that it is sufficiently small to leave a liquid absorption area 90 along the surface of the pad, for liquid acquisition, and sufficiently large to form a liquid barrier and prevent liquid flow from the liquid absorption area 90 towards the edges 53, 54. The part of the third sheet 30 extending along the lower surface 12 of the pad, preferably liquid acquisition material 15 and flaps 35, respectively 85 are connected together. This may for example be achieved by means of a connection which extends through the thickness of these layers. It is remarked that the connection will preferably not extend through the fold flaps 36, 37. Preferably the connection extends along the length of the pad in longitudinal direction. The fold flaps 36, 37 are double folded over the cover flaps 35, 85 respectively, along a fold which is closed at the position of the upper face 90 of the pad 1, and which is open towards upright longitudinal sides 6, 7, to form connection flaps 38, 39 along opposite longitudinal sides of the pad. Along opposite end parts of the third sheet in longitudinal direction 110, 120, 130, 140, as can be seen from figure 2, an end part of the third sheet may extend along and cover an outer face of the first and second sheet. At that position connection 53, 54 will extend through the third sheet 30, as well as through first and second sheets 10, 20 respectively.

The thus obtained pad, having the first, second and third sheets of liquid impermeable material 10, 20, 30 connected to it, is positioned on a fourth sheet 40 of liquid impermeable material, along the face of the first, second and third sheets of liquid impermeable material 10, 20, 30 which points away from the liquid absorbent material.

The fastening flaps 31, 32 of respectively the first and second sheet of liquid impermeable material 10, 20, are connected to the parts 43, 44 of the fourth sheet 40 which extend along upright cross sides 8, 9 of the pad 1. The connection is formed in thickness direction of the pad 1. This connection provides a form stable enveloping of the liquid absorbent material by sheets of liquid impermeable material. No connection to the garment is established. The inventors have namely observed that a connection along all the sides or edges of the pad shows a high risk to liquid leaks.

Flaps 38, 39 of the third sheet of liquid impermeable material 30 are connected to the parts of the fourth sheet 41, 42 which extend along the longitudinal upright sides 6, 7 and to the material of the garment 5 present at that position. When used with underpants, this will be a connection which extends along or forms the bows 78, 79.

## Claims

1. A garment comprising a moisture management incontinence pad (100) attached to the garment (5), wherein the pad (100) comprises:
(a) at least one sheet of a liquid absorbent material (1), with an inside surface (11) provided for residing nearest the body of a user of the garment and an outside surface (12) provided for residing nearest the garment (5), wherein the sheet of liquid absorbent material comprises a pair of opposite first and second upright end sides s (13, 14) which extend crosswise of the pad (100), and a pair of opposite first and second upright longitudinal sides (6, 7);
(b) a first (10) and a second (20) inside sheet of a liquid impermeable material, positioned at the opposite first and second opposite end sides (8, 9) of the pad (100), wherein a first end flap (23, 24) of each of the first and second inside sheet resides along a part of the inside surface (11) of the sheet of liquid absorbent material (1) which extends along respectively a first and second upright end sides (8, 9) of the pad (100) and is connected (2) to the liquid absorbent material (1) in thickness direction thereof, wherein the first end flap (23, 24) of each of the first and second inside sheet (10, 20) is connected to a second end flap (33, 34) of each of the first and second inside sheet (10, 20) crosswise of the pad, which second end flaps (33, 34) extend along at least part of the first end flap (23, 24) crosswise of the pad, wherein each of the second end flaps (33, 34) is connected to a fastening flap (31, 32) at a position (48, 49) opposite the connection to the first end flap (23, 24)
(c) at least one third sheet (30) of a liquid impermeable material, which extends in longitudinal direction of the pad and is positioned on a side of the liquid absorbent material (1) proximal to the garment, which third sheet of liquid impermeable material (30) is provided extend along at least part of the lower outside surface (12) and the opposite upright sides (6, 7) of the sheet of liquid absorbent material (1), and further along a first and second part of the inside surface (11) which extend along the opposite first and second upright sides (6, 7) of the sheet of liquid absorbent material by means of a first and second cover flap (35, 85), which first and second cover flaps (35, 85) are respectively covered by a first and second fold flap (36, 37) which extend over at least part of the first and second cover flaps (35, 85)) and which are connected to respectively a first and a second side fastening flap (38, 39) of the third sheet of liquid impermeable material (30) at a position (88, 89) opposite the connection to the first and second cover flaps (35, 85)
(d) a fourth outside sheet of a liquid impermeable material (40)), which is positioned on an outside face (35, 21, 22) of the first, second and third sheet of liquid impermeable material (10, 20, 30) which is provided to face the garment, opposite end sides of the fourth sheet (40) comprising a third and a fourth end fastening flap (43, 44) which run along respectively the first and second upright end sides (8, 9) of the liquid absorbent material, and which are connected to respectively the first and second end fastening flap (31, 32) of the first and second sheet of liquid impermeable material (10, 20) in thickness direction of the pad at a position beyond the sheet (1) of the liquid absorbent material, which fourth sheet (40) further comprises a first and a second side fastening flap (41, 42) which are connected to respectively the first and second side fasting flap (38, 39) of the third sheet (30) of liquid impermeable material in thickness direction of the pad at a position beyond the sheet of liquid absorbent material (1),
(e) wherein the first (38) and second (39) side fastening flap of the third liquid impermeable sheet 30 are respectively fastened to the first (41) and second (42) side fastening flap of the fourth liquid impermeable sheet and to the garment.

2. A garment as claimed in claim 1, wherein the first end flaps (33) of the first and second sheet (10, 20) of liquid impermeable material which extend along the inside surface (11) of the liquid absorbent material (1), and the second end fold flaps (34) are double folded thereover, and are connected along a fold, which is closed in the direction of the inside surface (11) of the sheet of liquid absorbent material.

3. A garment as claimed in claim 1 or 2, wherein the first and second cover flaps (35) of the third sheet (30) are connected to the first and second fold flap (36, 37) and are double folded thereover along a fold which is closed in the direction of the inside surface (11) of the sheet of liquid absorbent material.

4. An garment as claimed in any of the previous claims, wherein the first and second side fastening flap (41, 42) of the third sheet (30) of liquid impermeable material are provided for being fastened to respectively a first and second opposite side edge of a garment (51, 52) into which the moisture management incontinence pad is to be used.

5. A garment as claimed in any of the previous claims, wherein the first and second end fastening flap (31, 32) of respectively the first and second sheet of liquid impermeable material (10, 20) are provided to be in a floating arrangement with respect to the garment.

6. A garment as claimed in any of the previous claims, wherein the first (38) and second (39) side fastening flap of the third sheet (30) of liquid impermeable material are respectively connected to the first (41) and second (42) side fastening flap of the fourth sheet (40) of liquid impermeable material and to a side edge of a garment into which the pad is to be mounted.

7. A garment as claimed in any of the previous claims, wherein connecting is achieved through a technique selected from the group of stitching, braiding, tufting, needling, or a combination of two or more of these techniques.

8. A garment as claimed in any of the previous claims, wherein the first and second sheet of liquid impermeable material (10, 20) are made in one part.

9. A garment as claimed in any of the previous claims, wherein the liquid absorbent material (1) comprises several sheets of liquid absorbent material positioned on top of each other, which layers adhere to each other along at least part of their contact face.

10. A garment as claimed in any of the previous claims, wherein the inside surface (11) of the sheet of liquid absorbent material (1) provided for residing nearest the body of a user may be covered with a layer of a liquid acquisition material which permits liquid penetration into the liquid absorbing material and counteracts back flow of liquid from the pad towards and out of the liquid acquisition material.

11. A garment according to any of the previous claims, wherein the third sheet (30) and the fourth sheet (40) of liquid impermeable material are fastened to each other on opposite sides of the liquid permeable material (1) at a position (53, 54) adjacent to and beyond the upright side edges of the liquid permeable material (1).

12. A garment according to any of the previous claims, wherein the first and second sheet (10, 20) of liquid material are fastened to the fourth sheet (40) at a position (51, 52) adjacent to and beyond the upright end edges of liquid impermeable material.

13. A garment according to any of the previous claims, wherein upright sides of the liquid absorbent material (6 and 7) are enclosed by an envelope of a liquid impervious sheet at a position between the liquid absorbent material (11) and the upright extending parts of the third liquid impervious sheet (30), and wherein preferably upright sides (8, 9) are enclosed by an envelope at a position between the liquid absorbent material (11) and the upright extending parts of respectively the first and second liquid impervious sheet (10, 20).

14. A garment according to any of the previous claims, wherein the garment is an undergarment selected from the group of a sanitary towel, a nappy, a nursing pad bra liner, a female garment - for example panties, bikini underwear, thongs, G-strings, swimsuits, nightwear etc., a male undergarment - for example shorts, boxer shorts, boxer briefs, swimsuits, nightwear, etc.

15. A moisture management incontinence pad as part of the garment of any one of claims 1-14.
